(19) Europäisches Patentamt
European Patent Office
Office européen des brevets

(11) Veröffentlichungsnummer : **0 301 421 B1**

# (12) EUROPÄISCHE PATENTSCHRIFT

(45) Veröffentlichungstag der Patentschrift : **28.08.91 Patentblatt 91/35**

(51) Int. Cl.[5] : **C07C 321/00,** C07C 323/00, C07C 381/00, C07C 317/00, A61K 31/19, A61K 31/215

(21) Anmeldenummer : **88111808.7**

(22) Anmeldetag : **22.07.88**

## (54) Neue Schwefelverbindungen, Verfahren zu ihrer Herstellung sowie Arzneimittel.

(30) Priorität : **28.07.87 DE 3724923**

(43) Veröffentlichungstag der Anmeldung : **01.02.89 Patentblatt 89/05**

(45) Bekanntmachung des Hinweises auf die Patenterteilung : **28.08.91 Patentblatt 91/35**

(84) Benannte Vertragsstaaten : **AT BE CH DE ES FR GB GR IT LI LU NL SE**

(56) Entgegenhaltungen :
**GB-A- 780 520**
**GB-A- 790 199**
**US-A- 4 752 616**
**TETRAHEDRON LETTERS, Band 26, Nr. 23, 1985, Seiten 2787-2788 ; Pergamon Press Ltd, GB I.K. STAMOS : "Alkylation of aromatic compounds with pummerer rearrangement intermediates. Application to the preparation of methylaryl compounds."**

(73) Patentinhaber : **BOEHRINGER MANNHEIM GMBH**
**Sandhofer Strasse 116**
**W-6800 Mannheim 31 (DE)**

(72) Erfinder : **Reinholz, Erhard, Dr.**
**Kussmaulstrasse 9**
**W-6800 Mannheim 1 (DE)**
Erfinder : **Witte, Ernst-Christian, Dr.rer.nat.**
**Beethovenstrasse 2**
**W-6800 Mannheim 1 (DE)**
Erfinder : **Doerge, Liesel, Dr.med.**
**Schwalbenstrasse 26**
**W-6840 Lampertheim (DE)**
Erfinder : **Pill, Johannes Dr.Dr.Arzt.**
**In der Keitgasse 6**
**W-6906 Leimen 3 (DE)**
Erfinder : **Stegmeier, Karlheinz, Dr.rer.nat.**
**Kirchbergstrasse 17**
**W-6148 Heppenheim (DE)**

**EP 0 301 421 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft substituierte Sulfenyl-, Sulfinyl- und Sulfonylverbindungen und ihre Derivate, Verfahren zu ihrer Herstellung sowie Arzneimittel die diese enthalten der allgemeinen Formel I

$$\overset{(O)_m}{\overset{|}{R_1-S}}-B-W-A \qquad (I),$$

in welcher

- $R_1$ eine $C_1$-$C_6$-Alkyl- oder $C_2$-$C_6$-Alkenylgruppe, einen $C_3$-$C_7$-Cycloalkylrest, einen Aralkyl-, Aralkenyl- oder Arylrest, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_{16}$ Acyl oder Azid substituiert sein kann, und der Alkyl- oder Alkenylteil 1-5 bzw. 2-3 C-Atome aufweisen kann,
- m die Zahlen 0, 1 oder 2,
- B eine unverzweigte oder verzweigte, gesättigte Alkylenkette mit maximal 6 C-Atomen,
- W 1.2-, 1.3- oder 1.4-Phenylen,
- A einen Oxyessigsäurerest bedeuten

sowie deren pharmakologisch verträgliche Salze, Ester und Amide.

Weiterhin sind Gegenstand der Erfindung Verbindungen der Formel I, in welcher

- $R_1$ einen Aralkyl-, Aralkenyl- oder Arylrest, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_{16}$ Acyl oder Azid substituiert sein kann, und der Alkyl- oder Alkenylteil 1-5 bzw. 2-3 C-Atome aufweisen kann,
- m die Zahlen 0, 1 oder 2,
- B eine unverzweigte oder verzweigte, gesättigte Alkylenkette mit maximal 6 C-Atomen,
- W 1.2-, 1.3- oder 1.4-Phenylen,
- A eine Formyl-$C_1$-$C_5$-alkylgruppe, eine Hydroxy-$C_0$-$C_6$-alkylgruppe, einen Oxyessigsäurerest oder einen Rest D-$R_2$ darstellt, in dem D eine

$$\underset{O}{\underset{\|}{-C-}} \text{ oder } \underset{OH}{\underset{|}{-CH-}}\text{Gruppe}$$

und $R_2$ Wasserstoff, einen $C_1$-$C_5$ Alkyl-, einen Hydroxy-$C_1$-$C_5$-alkyl- oder einen Carboxy-$C_0$-$C_4$-alkylrest und gegebenenfalls deren Lactone bedeuten, sowie deren pharmakologisch verträglichen Salze, Ester und Amide.

Ebenfalls Gegenstand der Erfindung sind folgende Verbindungen :

4-[3-(2-Methylphenylsulfonyl)-propan-1-yl]-phenylessigsäure
4-[3-(2-Chlorphenylsulfenyl)-propan-1-yl]-phenylessigsäure
4-[3-(2-Chlorphenylsulfonyl)-propan-1-yl]-phenylessigsäure
4-[3-(2-Methylphenylsulfinyl)-propan-1-yl]-phenylessigsäure
4-[3-(3-Chlorphenylsulfinyl)-propan-1-yl]-phenylessigsäure
4-[3-(3-Chlorphenylsulfonyl)-propan-1-yl]-phenylessigsäure
4-[3-(3-Trifluormethylphenylsulfonyl)-propan-1-yl]-phenylessigsäure
4-[4-(2-Chlorphenylsulfinyl)-butan-1-yl]-phenylessigsäure
4-[4-(2-Chlorphenylsulfonyl)-butan-1-yl]-phenylessigsäure
4-[4-(3-Bromphenylsulfinyl)-butan-1-yl]-phenylessigsäure
4-[4-(3-Bromphenylsulfonyl)-butan-1-yl]-phenylessigsäure
4-[4-(2-Methylphenylsulfenyl)-butan-1-yl]-phenylessigsäure
4-[4-(2-Methylphenylsulfinyl)-butan-1-yl]-phenylessigsäure
4-[4-(2-Methylphenylsulfonyl)-butan-1-yl]-phenylessigsäure
4-[4-(3-Methylphenylsulfinyl)-butan-1-yl]-phenylessigsäure
4-[4-(4-Cyanophenylsulfonyl)-butan-1-yl]-phenylessigsäure

4-[4-(4-Methoxyphenylsulfenyl)-butan-1-yl]-phenylessigsäure
6-<4-[3-(4-Chlorphenylsulfenyl)-propan-1-yl]-phenyl>-hexansäure
6-<4-[3-(4-Chlorphenylsulfonyl)-propan-1-yl]-phenyl>-hexansäure

Diese Verbindungen werden auch von der allgemeinen Formel der nicht vorveröffentlichten EP-A-297 768 umfaßt, sind aber namentlich dort nicht aufgeführt.

Die neuen Verbindungen der allgemeinen Formel I sowie die neuen Einzelverbindungen zeigen eine ausgezeichnete antagonistische Wirkung gegenüber Thromboxan $A_2$ sowie gegen Prostaglandin-endoperoxide. Sie inhibieren die Aggregation von Blutplättchen und verhindern die Konstriktion der glatten Muskulatur sowie die Bronchokonstriktion. Sie sind außerdem wertvolle Heilmittel zur Behandlung pathologischer Veränderungen der Nierenfunktion. Ferner erweisen sie sich als wirksame Lipidsenker.

Diese Eigenschaften machen sie zu wertvollen Heilmitteln zur Behandlung z.B. von cardiovaskulären Erkrankungen und von Asthma und zur Prophylaxe einer Schocklunge. Sie können weiterhin verwendet werden bei Organtransplantationen und Nierendialyse und sind geeignet, Rezidive bei Magengeschwüren zu verhindern. Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können z.B. gegen cerebrale ischaemische Zustände eingesetzt werden.

Ist $R_1$ eine Alkyl- oder eine Alkenylgruppe, so seien darunter unverzweigte oder verzweigte Gruppen mit 1-6 bzw. 2-6 C-Atomen verstanden. Bevorzugt sind die Methyl-, Ethyl-, Butyl-, Hexyl-, Butenyl- und die Pentenylgruppe.

Als Cycloalkylrest für $R_1$ kommen beispielsweise die Cyclopropyl-, Cyclopentyl- und Cyclohexylreste in Frage.

Als "Arylrest", allein oder in Verbindung mit einer Alkyl-oder Alkylenkette, sind in allen Fällen aromatische Kohlenwasserstoffe mit 6-14 C-Atomen, insbesondere der Phenyl-, der Biphenyl-, der Naphthyl- und der Fluorenylrest zu verstehen. Diese Arylreste können in allen möglichen Positionen ein- oder mehrfach substituiert sein, wobei als Substituenten Halogen, $C_1$-$C_6$ Alkyl, $C_1$-$C_6$ Alkoxyl, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_6$ Acyl und Azid in Frage kommen.

Unter Halogen sei in allen Fällen Fluor, Chlor und Brom zu verstehen.

Bevorzugte Substituenten sind beispielsweise die Methoxy-, Ethoxy-, Methyl-, Ethyl-, Propyl-, tert.-Butyl-, Acetylamino-, Acetyl-, Methylamino-, Dimethylamino-, Ethylamino- und Diethylaminogruppe.

Als Aralkylreste $R_1$ kommen solche in Frage, deren geradkettiger oder verzweigter Alkylenanteil 1-5 Kohlenstoffatome enthält. Bevorzugte Aralkylreste $R_1$ sind die ggf. substituierten Reste Benzyl bzw. Phenethyl.

Unter Aralkenylresten $R_1$ sind solche zu verstehen, deren Alkenylanteil 2-3 Kohlenstoffatome enthält. Hier sind bevorzugt der Cinnamyl- und der 4-Chlorcinnamylrest.

m bedeutet die Zahl 0, 1 oder 2.
B bedeutet eine 1-6 C-Atome enthaltende Alkylenkette. Bevorzugt sind Ketten, mit 1-5 C-Atomen.
A wird definiert als eine Oxyessigsäuregruppe oder als Alkylkette mit 1-6 C-Atomen, die durch Sauerstoff enthaltende Funktionen wie Oxo- oder Hydroxygruppen und gegebenenfalls zusätzliche Carboxylgruppen substituiert sein kann.

Bevorzugt sind die nachstehend aufgeführten Gruppen A :

1. A = eine unverzweigte oder verzweigte, gesättigte Alkylkette, die endständig eine Hydroxygruppe trägt, insbesondere solche der Formel

$$-(CH_2)_p\text{-}OH$$

in welcher p die Zahlen 1 bis 6 darstellen kann

2. A = eine Acylgruppe $-CO\text{-}R_2$, wobei
   a) $R_2$ = H (Formylrest) oder
   b) $R_2$ = ein unverzweigter oder verzweigter, gesättigter Alkylrest mit 1-5 C-Atomen ; bevorzugt sind Methyl, Ethyl, n-Propyl und n-Butyl.
   c) $R_2$ = eine unverzweigte oder verzweigte Alkylkette mit endständiger Carboxyl funktion, wobei bevorzugt sind Verbindungen der Formel

$$R_2 = -(CH_2)_{p-2}\text{-}COOH$$

in welcher p die Zahlen 2 bis 6, insbesondere aber 4 bis 6 darstellen kann.

3. A = eine Gruppe

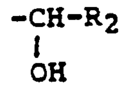

wobei $R_2$ die oben angegebenen Bedeutungen hat.

Für den Fall, daß $R_2$ ein wie unter 4 definierter Alkylrest ist, seien auch hier Methyl, Ethyl, n-Propyl und n-Butyl bevorzugt.

Ist $R_2$ dagegen eine Alkylgruppe mit endständiger Hydroxygruppe, so sind bevorzugt Verbindungen mit

$$R_2 = -(CH_2)_{p-1}\text{-}OH$$

wobei p = 3, 4 oder 5 bedeutet.

Stellt $R_2$ eine Alkylgruppe mit endständiger Carboxylfunktion dar, so werden bevorzugt solche Verbindungen, in denen

$$R_2 = -(CH_2)_{p-2}\text{-}COOH$$

bedeutet, wobei p die Werte 4 oder 5 hat.

4. A = eine Gruppe $-OCH_2COOH$.

In allen Fällen soll der Begriff "Carboxylfunktion" auch Ester und Amide solcher Carbonsäuren einschließen.

Als Ester kommen solche mit niederen einwertigen Alkoholen (wie z.B. Methanol oder t-Butanol) oder mit mehrwertigen Alkoholen (wie z.B. Glycerin) infrage, es seien aber auch solche Alkohole eingeschlossen, die noch andere funktionelle Gruppen enthalten, wie z.B. Ethanolamin.

Beansprucht werden auch die "inneren Ester" der Hydroxy-carbonsäuren geeigneter Kettenlängen, die Lactone. Für den Fall, daß die Funktion A asymmetrische Kohlenstoffatome enthält, so seien sowohl die reinen optischen Isomeren als auch deren Gemische/Racemate umfaßt. In allen Fällen in denen C-C-Doppelbindungen auftreten, werden hier die reinen E- und Z-Isomeren sowie auch deren Gemische beansprucht.

Die Herstellung der Verbindungen der allgemeinen Formel I mit m = 0 (d.h. die Herstellung der Sulfenylverbindungen) ist dadurch gekennzeichnet, daß man

a) eine Verbindung der allgemeinen Formel II,

X-B-W-A (II),

in welcher B, W und A die oben angegebene Bedeutung haben und X hier und in allen anderen Fällen einen reaktiven Rest wie z.B. Halogen, oder einen Ester einer Sulfonsäure, wie z.B. Tosylat oder Mesylat darstellt, in an sich bekannter Weise mit einem Mercaptan der allgemeinen Formel III

$R_1$-SH (III),

in welcher $R_1$ die oben angegebene Bedeutung hat, umsetzt. Für den Fall, daß A Hydroxygruppen enthalten soll, verwendet man vorteilhaft eine solche Verbindung II, die an der Stelle der Hydroxygruppe eine Oxogruppe oder eine Esterfunktion (oder ggf. beide) enthält. Diese Gruppen werden im Anschluß an die erfolgte Reaktion zur Hydroxyfunktion reduziert.

a1) Umgekehrt kann man für den Fall, daß $R_1$ einen Alkyl,-Alkenyl-, Cycloalkyl- oder Aralkylrest darstellt, ein Mercaptan der allgemeinen Formel IV

HS-B-W-A (IV),

in an sich bekannter Weise mit einer Verbindung der allgemeinen Formel V

$R_1$-X (V),

wobei A, B, $R_1$, W und X die weiter oben genannten Bedeutungen haben, umsetzen.

a2) Die Sulfenylverbindungen können auch durch Umsetzung von Verbindungen mit m = 1 oder 2 mit

geeigneten Reduktionsmitteln erhalten werden.

b) Die Herstellung der Verbindungen der allgemeinen Formel I mit m = 1 (d.h. die Herstellung der Sulfinylverbindungen) erfolgt durch Umsetzung der Verbindungen der allgemeinen Formel I mit m = 0 mit geeigneten Oxidationsmitteln.

Sie kann auch durch Umsetzung von Verbindungen mit m = 2 mit geeigneten Reduktionsmitteln erfolgen.

c) Die Herstellung der Verbindungen der allgemeinen Formel I mit m = 2 (d.h. die Herstellung der Sulfonylverbindungen) erfolgt entweder

c1) durch Oxidation der Verbindungen der allgemeinen Formel I mit m = 0 mit geeigneten Oxidationsmitteln

oder

c2) durch Weiteroxidation der Verbindungen der allgemeinen Formel I mit m = 1 mit geeigneten Oxidationsmitteln.

c3) Eine weitere Möglichkeit zur Herstellung der Verbindungen der allgemeinen Formel I mit m = 2 besteht darin, daß man eine Sulfensäure der allgemeinen Formel VI

$$R_1\text{-SO-OH} \quad \text{(VI)},$$

in der $R_1$ die weiter oben genannten Bedeutung hat, oder eines ihrer Salze mit einer Verbindung der allgemeinen Formel II zur Reaktion bringt. Für den Fall, daß A Hydroxygruppen enthalten soll, verwendet man vorteilhaft eine solche Verbindung II, die an der Stelle der Hydroxy gruppe eine Oxogruppe oder eine Esterfunktion (oder ggf. beide) enthält. Diese Gruppen werden im Anschluß an die erfolgte Reaktion zur Hydroxyfunktion reduziert.

c4) Desweiteren kann man Verbindungen der allgemeinen Formel I in der m = 2 ist, durch Umsetzung von Verbindungen der allgemeinen Formel II, in der X = $SO_2$Hal ist, mit geeigneten Aromaten erhalten.

d) Zum Aufbau der Verbindungen der allgemeinen Formel I, in der W o, m oder p-substituiertes Phenylen bedeutet, kann man Verbindungen der allgemeinen Formel VII

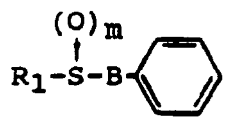

(VII),

in der $R_1$, B und m die bereits genannten Bedeutungen haben, in an sich bekannter Weise mit Verbindungen der allgemeinen Formel VIII, bzw. VIII a

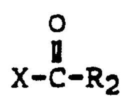

(VIII),

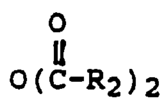

(VIII a)

in der X Halogen ist und $R_2$ die weiter o.a. Bedeutung hat, umsetzen.

e) Desweiteren können die Verbindungen der allgemeinen Formel I, in der A eine $OCH_2$-COOH-Gruppe darstellt, durch Umsetzung von Verbindungen der allgemeinen Formel IX

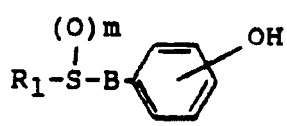

(IX),

in der $R_1$, B und m die o.a. Bedeutung haben, mit Chlor- oder Bromessigsäure bzw. ihren Estern hergestellt werden.

Für den Fall, daß A eine Carbonsäuregruppe enthalten soll, verwendet man vorteilhaft einen Ester dieser Säure. Diese Ester können im Anschluß an die erfolgte Reaktion wieder verseift werden.

Die gewünschten Verbindungen der allgemeinen Formel I können auch dadurch erhalten werden, daß man funktionelle Gruppen umwandelt.

a) Zur Einführung von Hydroxygruppen in den Rest A kommen folgende Verfahren in Frage :

1. Reduktion einer Carbonylgruppe
2. Reduktion einer Carbonsäure- oder einer Carbonsäureester-funktion.
3. Gleichzeitige Reduktion beider.

b) Die Einführung von Doppelbindungen in den Rest A erfolgt, indem man aus einer in A enthaltenen Gruppe (X)

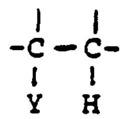 (X),

in welcher Y Halogen oder Hydroxyl oder eine funktionell abgewandelte Hydroxylgruppe darstellt, HY abspaltet.

b1) Eine weitere Möglichkeit besteht in der Umsetzung von Oxoverbindungen mit geeigneten phosphororganischen Verbindungen im Sinne (modifizierter) Wittig-Reaktionen. Als Ausgangsmaterial dienen in diesem Falle Verbindungen der allgemeinen Formel II, in denen

a) A = 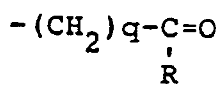

wobei q die Zahlen 0-5 und R Wasserstoff oder Alkyl darstellen, bzw.

β) A = $-(CH_2)q-PO(O-Alk)_2$

wobei q die Zahlen 1-5 darstellen, bzw.

γ) A = $-(CH_2)q-PR_3Hal^-$

wobei q die Zahlen 1-5 und R z.B. Phenyl darstellen, bedeuten.

c) Durch Hydrieren der nach b) bzw. b1) erhaltenen Verbindungen mit ungesättigtem Rest A erhält man analoge Verbindungen mit gesättigter Kohlenstoffkette. Derartige Verbindungen lassen sich auch durch Reduktion von in A enthaltenen Ketogruppen darstellen.

Die Umsetzung der Mercaptane der allgemeinen Formel III mit Verbindungen der allgemeinen Formel II bzw. der Mercaptane der allgemeinen Formel IV mit Verbindungen der allgemeinen Formel V erfolgt zweckmäßig unter Zusatz eines säurebindenden Mittels, wie z.B. einem Alkalicarbonat, einem Alkalihydroxid, einem Alkoholat eines niederen aliphatischen Alkohols oder einem Alkalihydrid.

Als Reaktionsmedium kann ein niederer aliphatischer Alkohol, ein niederes aliphatisches Keton oder ein polares aprotisches Lösungsmittel wie DMF dienen.

Die Oxidation der Sulfenylverbindungen zu den Sulfinyl-bzw. Sulfonylverbindungen erfolgt entweder mit einem anorganischen Oxidationsmittel wie $H_2O_2$, $NaJO_4$, $KMnO_4$, NaClO oder Oxon® (Kaliumhydrogenperoxomonosulfat), oder mit einer organischen Persäure wie z.B. m-Chlorperbenzoesäure.Als Lösungsmittel kommen Wasser, niedere aliphatische Carbonsäuren wie z.B. Essigsäure, oder chlorierte Kohlenwasserstoffe in Betracht.

Die Herstellung der Sulfinylverbindungen erfolgt durch Zugabe von einem Äquivalent des betreffenden Oxidationsmittels zur Sulfenylverbindung.

Zur Vermeidung der Bildung der Sulfonylverbindungen kann auch mit Sauerstoff in Gegenwart von Cerammonitrat in Acetonitril, unter Bedingungen wie sie von Riley et al. J. Chem. Soc. Chem. Comm. 1986, 1097 angegeben werden, oxidiert werden.

Vorteilhaft ist auch die Oxidation mittels Oxon in Methylenchlorid in Gegenwart eines Phasentransfer-Katalysators in Analogie zu Evans et al. Synth. Comm. 16, 1207 (1986).

Die Herstellung der Sulfonylverbindungen erfolgt durch Zugabe von zwei Äquivalenten des Oxidationsmittels zur Sulfenylverbindung oder von einem Äquivalent zur Sulfinylverbindung.

Zur Alkylierung der Sulfensäuren der allgemeinen Formel VI führt man diese zweckmäßig in ihr Alkalisalz über und bringt sie mit Verbindungen der allgemeinen Formel II in einem niederen aliphatischen Alkohol, einem niederen aliphatischen Keton oder DMF zur Reaktion.

Die Umsetzung von Sulfonylhalogeniden mit Aromaten erfolgt unter Zusatz einer Lewis-Säure, z.B. von

$AlCl_3$, z.B. in $CS_2$, oder der Aromat selber dient als Lösungsmittel.

Die Umsetzung der Verbindungen der allgemeinen Formel VII mit den Verbindungen der allgemeinen Formel VIII bzw. VIII a erfolgt unter Zusatz einer Lewissäure wie z.B. $AlCl_3$ in einem aprotischen Lösungmittel wie z.B. $CS_2$ oder $Cl_2CH$-$CHCl_2$.

Die Alkylierung der Verbindungen mit der allgemeinen Formel IX mit Chlor- bzw. Bromessigsäure bzw. ihren Estern erfolgt vorteilhaft unter Zusatz eines säurebindenden Mittels wie etwa einem Alkalihydroxid, einem Alkalicarbonat, einem Alkalihydrid oder einem Alkoholat eines niederen aliphatischen Alkohols. Als Reaktionsmedium kann Wasser, ein niederer aliphatischer Alkohol, ein niederes aliphatisches Keton oder ein polares aprotisches Lösungsmittel wie z.B. DMF dienen.

Die im Anschluß an die Bildung der Sulfenyl-, Sulfinyl- oder Sulfonylverbindungen möglichen Umwandlungen im Rest A lassen sich wie folgt beschreiben :

Zur Umwandlung einer Carbonylgruppe in eine Hydroxygruppe sind hier alle gängigen Reduktions-Verfahren einsetzbar. Bevorzugt ist die Reduktion mit komplexen Borhydriden, z.B. mit Natriumborhydrid, wobei protische Lösungsmittel wie Wasser, (wässrige) Alkohole oder wässriges Dioxan als Reaktionsmedium dienen. Bei Abwesenheit anderer reduzierbarer Gruppen kann die Reduktion auch mit komplexen Aluminiumhydriden wie $LiAlH_4$ oder Dibal (Diisobutylaluminiumhydrid) durchgeführt werden, wobei hier aprotische Lösungsmittel wie Ether, THF oder Dioxan als Reaktionsmedium dienen. Die Carbonylreduktion kann aber auch mit katalytisch angeregtem Wasserstoff erfolgen, z.B. mit $H_2$/Raney-Nickel oder durch Umsetzen mit Nickel-Aluminium-Legierung in wässrigem Alkali.

Zur Reduktion der Carboxylfunktion sind alle für diesen Zweck üblichen Reduktionsmittel geeignet, z.B. komplexe Hydride wie Lithiumaluminium-hydrid oder Dibal, oder Boran-addukte wie z.B. $BH_3$. THF. Die Reduktion kann aber auch vorteilhaft durch Reduzieren eines Derivates der Carbonsäure, z.B. eines gemischten Anhydrids aus der Carbonsäure und einem Kohlensäurehalbester, erfolgen. Als Reduktionsmittel verwendet man hier bevorzugt komplexe Borhydride wie z.B. $NaBH_4$ in protischen Lösungsmitteln.

Zur Reduktion geeignete Derivate der Carbonsäuren sind z.B. auch deren Ester, welche sich nach literaturüblichen Methoden zu primären Alkoholen umsetzen lassen. Bevorzugte Reduktionsmittel sind auch hier komplexe Aluminiumhydride wie z.B. Lithiumalanat oder Dibal.

Soll die Carboxylfunktion reduziert werden, ohne daß eine gleichzeitig in A befindliche Oxogruppe mitreduziert wird, so ist letztere z.B. durch Ketalisierung intermediär zu schützen. Solche Hydroxy-ketone sind auch darstellbar, indem man sowohl die Ketogruppe als auch die Carboxylfunktion reduziert (wobei man die ebenfalls beanspruchten Diole erhält) und anschließend die sekundäre Hydroxyfunktion selektiv zur Ketofunktion oxidiert. Hierzu geeignet ist z.B. aktives Mangandioxid.

Die Reduktion der Sulfonyl- bzw. der Sulfinylverbindungen zu den Sulfinyl- bzw. Sulfenylverbindungen ist mit allen üblichen Reduktionsmitteln möglich, wie z.B. $LiAlH_4$, Diisobutylaluminiumhydrid, Boranen, Silanen, Zinnhydriden, $SnCl_2$/HCl und 3-wertigen Phosphorreagenzien. Die Reduktion kann aber auch mit Wasserstoff in Gegenwart eines Katalysators, wie z.B. Palladium oder durch Erhitzen mit elementarem Schwefel erfolgen.

Zur Überführung der Ester in die Carbonsäuren kommen alle gängigen Verseifungsverfahren in Betracht: Säurekatalysierte Verseifung, wobei die Säure eine organische oder anorganische sein kann, oder basenkatalysierte Verseifung mit einem Alkylihydroxyd-, carbonat oder -hydrogencarbonat.

Zur Einführung von Doppelbindungen in den Rest A eignen sich alle gängigen Eliminierungsreaktionen :

Abspaltung von Wasser aus Hydroxyverbindungen (säurekatalysiert, durch Erwärmen z.B. mit Schwefelsäure oder 85proz. Phosphorsäure) ; Abspaltung von Halogenwasserstoff aus Halogeniden und von Sulfonsäuren aus Sulfonyloxyverbindungen wie z.B. Tosyloxy mittels starker Basen wie Kalium-tert.-butanolat oder DBU (Diazabicycloundecan), und schließlich die Abspaltung von Essigsäure aus Acetoxyverbindungen und von Xanthogensäure aus Xanthogenaten durch Erhitzen. Zum Aufbau von Gruppen A, die eine Doppelbindung enthalten, eignet sich auch die Kondensation von zwei Komponenten, deren eine eine Oxogruppe, die andere dagegen eine Phosphoniumsalzgruppe (Wittig-Reaktion) oder eine Phosphat-Gruppe (Wittig-Horner-Emmons-Reaktion) enthalten. Diese Reaktionen können in verschiedensten Lösungsmitteln wie z.B. Wasser, Methanol, DMF, Ethylenglykol oder Glykolethern durchgeführt werden und verlaufen in Gegenwart von Basen wie z.B. Natriumcarbonat, Natriumalkoholaten, Natriumhydrid oder Butyllithium in den jeweils geeigneten Lösungsmitteln, wobei je nach Reaktionsbedingungen cis- oder trans- Isomere oder Gemische beider entstehen können.

Zur Herstellung von Verbindungen, in denen A eine gesättigte Alkylkette ist, kommt man u.a. durch Hydrierung von olefinischem A. Die Hydrierung wird vorzugsweise bei Normaldruck oder bei erhöhtem Druck in Gegenwart von Metallkatalysatoren wie z.B. Palladium oder Platin in Lösungsmitteln wie z.B. Essigsäure oder in niederen Alkoholen vorgenommen. Weiterhin bevorzugt ist die Reduktion von Verbindungen, die im Rest A eine Hydroxy- oder eine Oxogruppe enthalten. Die Reduktion von Hydroxygruppen enthaltenden Verbindungen geschieht in Gegenwart starker Säuren wie z.B. einer Spur Perchlorsäure mittels Wasserstoff mit Hilfe von Palladium- oder Platinkatalysator. Zur Reduktion einer Oxogruppe eignen sich zahlreiche Verfahren. Die Reduktion kann z.B. nach Clemmensen mittels Zink/Salzsäure erfolgen, oder man bildet aus dem Keton zunächst ein Tosylhydrazon und reduziert dieses. Bevorzugt ist aber auch hier die Reduktion mittels katalytisch angeregtem Wasserstoff unter den oben angegebenen Bedingungen.

Eine bereits im Molekül befindliche Alkanoylfunktion kann man in eine Alkylcarbonsäurekette gleicher Kohlenstoffzahl umwandeln, indem man sie den Bedingungen der Willgerodt- oder Willgerodt-Kindler-Reaktion unterwirft, d.h. mit Morpholin und Schwefel umsetzt und anschließend das entstandene Thiomorpholid hydrolysiert. So kann man z.B. eine substituiertes Acetophenon in eine subst. Phenylessigsäure umwandeln.

Eine andere Möglichkeit zur Umwandlung einer Acetylgruppe in eine Essigsäure besteht in der Oxidation mit $Tl(NO_3)_3$.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg Tag in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Methyl-, Ethyl- und tert. Butyl-Estern und Amiden die folgenden :

2-[2-(4-Chlorphenylsulfonyl)-ethan-1-yl]-phenoxyessigsäure
2-[4-(4-Chlorphenylsulfonyl)-butan-1-yl]-phenoxyessigsäure
3-[3-(Phenylsulfonyl)-propan-1-yl]-phenoxyessigsäure
3-[3-(4-Chlorphenylsulfonyl)-propan-1-yl]-phenoxyessigsäure
2-[3-(4-Chlorphenylsulfonyl)-propan-1-yl]-phenoxyessigsäure
3-[4-(Phenylsulfonyl)-butan-1-yl]-phenoxyessigsäure
3-[4-(4-Chlorphenylsulfonyl)-butan-1-yl]-phenoxyessigsäure
3-[3-(4-Bromphenylsulfonyl)-propan-1-yl]-phenoxyessigsäure
3-[4-(4-Bromphenylsulfonyl)-butan-1-yl]-phenoxyessigsäure
4-[3-(4-Chlorphenylsulfonyl)-propan-1-yl]-phenylhexansäure
4-[3-(4-Chlorphenylsulfonyl)-propan-1-yl)-phenylethanol
4-[3-(Methylsulfonyl)-propan-1-yl]-phenoxyessigsäure
4-[3-(4-Chlorphenethylsulfonyl)-propan-1-yl]-phenoxyessigsäure
4-[3-(4-Phenylphenylsulfonyl)-propan-1-yl]phenoxyessigsäure
4-Hydroxy-4-[3-(4-Chlorphenylsulfonyl)-propan-1-yl]phenylbutanol
4-Hydroxy-4-[3-(4-Chlorphenylsulfonyl)-propan-1-yl]-phenylbuttersäure

## Beispiel 1

42 g (0.17 mol) 4-(3-Chlor-1-oxo-propan-1-yl)-phenylessigsäureethylester wurden in 400 ml Ethanol mit 1% Pd auf A-Kohle bei Raumtemperatur und Normaldruck hydriert. Nach Absaugen des Katalysators und Eindampfen wurde der Rückstand in Essigester aufgenommen und mit $NaHCO_3$-Lsg. und verd. HCl ausgeschüttelt. Anschließend wurde neutral gewaschen, getrocknet und eingedampft. Es wurden 37 g (Ausbeute : 93% d.Th.) 4-(3-Chlorpropyl)-phenylessigsäureethylester als Öl erhalten.

1.15 g (50 mmol) Natrium wurden in 50 ml Ethanol gelöst und mit 6.2 g (50 mmol) 2-Methylthiophenol versetzt. Es wurde dann 10 min unter Rückfluß gekocht und nach dem Abkühlen 11.4 g (50 mmol) 4-(3-Chlorpropyl)-phenylessigsäureethylester zugetropft. Anschließend wurde 20 Stunden bei Raumtemperatur gerührt, dann eingedampft und der Rückstand in Essigester aufgenommen. Es wurde 3 mal mit Wasser ausgeschüttelt, getrocknet und eingedampft.

Es wurden 7.4 g (Ausbeute : 45% d.Th.) 4-[3-(2-Methylphenylsulfenyl)-propan-1-yl]-phenylessigsäureethylester als Öl erhalten :

Aus 4-(3-Chlorpropyl)-phenylessigsäureethylester und dem entsprechenden Thiophenol wurden analog hergestellt :

1) 4-[3-(2-Chlorphenylsulfenyl)-propan-1-yl]-phenylessigsäureethylester
Ausbeute : 24% d.Th. ; Öl.

2) 4-[3-(3-Chlorphenylsulfenyl)-propan-1-yl]-phenylessigsäureethylester
Ausbeute : 52% d.Th. ; Öl.

Aus 4-(4-Chlorbutyl)-phenylessigsäureethylester und dem entsprechenden Thiophenol wurden analog erhalten :

3) 4-[4-(4-Methoxyphenylsulfenyl)-butan-1-yl]-phenylessigsäureethylester
Ausbeute : 81% d.Th. ; Öl.

4) 4-[4-(3-Methylphenylsulfenyl)-butan-1-yl]-phenylessigsäureethylester
Ausbeute : 69% d.Th. ; Öl.

5) 4-[4-(2-Methylphenylsulfenyl)-butan-1-yl]-phenylessigsäureethylester
Ausbeute : 75% d.Th. ; Öl.

6) 4-[4-(2-Chlorphenylsulfenyl)-butan-1-yl]-phenylessigsäureethylester
Ausbeute : 91% d.Th. ; Öl.

7) 4-[4-(3-Bromphenylsulfenyl)-butan-1-yl]-phenylessigsäureethylester
Ausbeute : 73% d.Th. ; Öl.

8) 4-<4-[3-(4-Chlor-phenylsulfenyl)-propan-1-yl]-phenyl>-4-oxo-butansäure-methylester
Ausbeute : 63% d.Th. ; Fp. 121°C

und aus 4-[4-(4-Brombutyl)-phenyl]-4-oxo-butansäuremethylester :

9) 4-<4-[4-(4-Chlor-phenylsulfenyl)-butan-1-yl]-phenyl>-4-oxo-butansäure-methylester
Ausbeute : 39% d.Th. ; Öl.

und aus 6-[4-(3-Chlorpropyl)-phenyl]-hexansäure-methylester wurde analog hergestellt :

10) 6-<4-[3-(4-Chlor-phenylsulfenyl)-propan-1-yl]phenyl>-hexansäure-methylester
Ausbeute : 75% d.Th. ; Schmp. 40-41°C (Ethanol).

## Beispiel 2

### 4-[3-(2-Methylphenylsulfinyl)-propan-1-yl]-phenylessigsäureethylester

6.9 g (21 mmol) 4-[3-(2-Methylphenylsulfenyl)-propan-1-yl]-phenylessigsäureethylester wurden in 50 ml Eisessig gelöst, mit 1.53 ml 30proz. $H_2O_2$ versetzt und 2 Tage bei Raumtemperatur gerührt. Anschließend wurde mit etwas Platinmohr versetzt, 2 Stunden bei Raumtemperatur gerührt, abfiltriert und eingedampft. Es wurden 5.3 g (Ausbeute : 73% d.Th.) der Titelverbindung als Öl erhalten.

Aus den entsprechenden Sulfenylverbindungen wurden analog hergestellt :

2) 4-[3-(2-Chlorphenylsulfinyl)-propan-1-yl]-phenylessigsäureethylester
Ausbeute : 55% d.Th. ; Öl,

3) 4-[3-(3-Chlorphenylsulfinyl)-propan-1-yl]-phenylessigsäureethylester
Ausbeute : 47% d.Th. ; Öl.

4) 4-[4-(4-Methoxyphenylsulfinyl)-butan-1-yl]-phenylessigsäureethylester
Ausbeute : 32% d.Th. ; Öl.

5) 4-[4-(3-Methylphenylsulfinyl)-butan-1-yl]-phenylessigsäureethylester
Ausbeute : 97% d.Th. ; Öl,

6) 4-[3-(4-(2-Methylphenylsulfinyl)-butan-1-yl]-phenylessigsäureethylester
Ausbeute : 77% d.Th. ; Öl.

7) 4-[4-(2-Chlorphenylsulfinyl)-butan-1-yl]-phenylessigsäureethylester
Ausbeute : 60% d.Th. ; Öl.

8) 4-[4-(3-Bromphenylsulfinyl)-butan-1-yl]-phenylessigsäureethylester
Ausbeute : 82% d.Th. ; Öl.

Beispiel 3

4-[3-(2-Methylphenylsulfonyl)-propan-1-yl]-phenylessigsäureethylester

7.5 g (23 mmol) 4-[3-(2-Methylphenylsulfenyl)-propan-1-yl]-phenylessigsäureethylester wurden in 50 ml Eisessig gelöst, mit 5 ml 30proz. $H_2O_2$ versetzt und bei Raumtemperatur gerührt. Nach 24 Stunden wurden weitere 5 ml 30proz. $H_2O_2$ zugegeben und nochmals 48 Stunden bei Raumtemperatur gerührt. Anschließend wurde etwas Platinmohr zugegeben, 2 Stunden bei Raumtemperatur gerührt, filtriert und eingedampft. Es wurden 6.0 g (Ausbeute : 72% d.Th.) als Öl erhalten.

Aus den entsprechenden Sulfenylverbindungen wurden analog erhalten :

1) 4-[3-(2-Chlorphenylsulfonyl)-propan-1-yl]-phenylessigsäureethylester
Ausbeute : 50% d.Th. ; Öl.

2) 4-[3-(3-Chlorphenylsulfonyl)-propan-1-yl]-phenylessigsäureethylester
Ausbeute : 44% d.Th. ; Öl.

3) 4-[4-(2-Methylphenylsulfonyl)-butan-1-yl]-phenylessigsäureethylester
Ausbeute : 93% d.Th. ; Öl.

4) 4-[4-(2-Chlorphenylsulfonyl)-butan-1-yl]-phenylessigsäureethylester
Ausbeute : 67% d.Th. ; Öl.

5) 4-[4-(3-Bromphenylsulfonyl)-butan-1-yl]-phenylessigsäureethylester
Ausbeute : 71% d.Th. ; Öl.

6) 4-<4-[3-(4-Chlorphenylsulfonyl)-propan-1-yl]-phenyl>-4-oxo-butansäure-methylester
Ausbeute : 92% d.Th. ; Öl.

7) 4-<4-[4-(4-Chlorphenylsulfonyl)-butan-1-yl]-phenyl>-4-oxo-butansäure-methylester
Ausbeute : 71% d.Th. ; Öl.

8) 6-<4-[3-(4-Chlorphenylsulfonyl)-propan-1-yl]-phenyl>-hexansäure-methylester
Ausbeute : 91% d.Th. ; Fp : 60-61°C (Ethanol).

Beispiel 4

4-[3-(Phenylsulfonyl)-propan-1-yl]-phenylessigsäure

3.44 g (12 mMol) 4-[3-(Phenylsulfenyl)-propan-1-yl]-phenylessigsäure wurden in 24 ml Eisessig gelöst, mit 3.72 ml 30proz. $H_2O_2$ versetzt und 20 Stunden bei Raumtemperatur gerührt. Anschließend wurde mit etwas Platinmohr versetzt und noch 1 Stunde bei Raumtemperatur gerührt. Durch Erwärmen wurde die ausgefallene Substanz in Lösung gebracht und das Platinmohr abfiltriert. Nach Abziehen des Eisessigs wurde der Rückstand unter Erwärmen in Essigester gelöst und filtriert. Das erhaltene Filtrat wurde bis zur leichten Trübung mit Ligroin versetzt und die ausgefallene Substanz abfiltriert. Es wurden 2.6 g (68% d.Th.) der Titelverbindung vom Schmelzpunkt 141-142°C erhalten.

In Analogie dazu wurden aus den entsprechenden Sulfenyl-carbonsäuren erhalten :

2) 4-[4-(4-Methoxyphenylsulfonyl)-butan-1-yl]-phenylessigsäure
Ausbeute : 66% d.Th. ; Öl.

Beispiel 5

4-[3-(3-Trifluormethylphenylsulfonyl)-propan-1-yl]-phenylessigsäureethylester

8.1 g (35 mmol) 3-Trifluormethylphenylsulfinsäure-Natriumsalz wurden in 50 ml DMF gelöst und mit einer Lösung von 9.43 g (39.2 mmol) 4-(3-Chlorpropyl)-phenylessigsäureethylester in 50 ml DMF versetzt. Dann wurde 6 Stunden bei 100°C gerührt. Anschließend wurde das DMF abgezogen, der Rückstand in Essigester aufgenommen, 3 mal mit verd. NaOH ausgeschüttelt, neutral gewaschen, getrocknet und eingedampft. Nach Reinigung über eine Kieselgelsäule im Laufsystem Ligroin/Ether (15 : 10) wurden 2.3 g (16% d.Th.) der Titelverbindung als Öl erhalten.

Aus der entsprechenden Sulfinsäure und dem entsprechenden Halogenid wurde analog erhalten :

2) 4-[4-(4-Cyanphenylsulfonyl)-butan-1-yl]-phenylessigsäureethylester
Ausbeute : 30% d.Th. ; Öl.

Beispiel 6

4-[3-(2-Methylphenylsulfonyl)-propan-1-yl]-phenylessigsäure

14.4 g (40 mmol) 4-[3-(2-Methylphenylsulfonyl)-propan-1-yl]-phenylessigsäureethylester wurden in 30 ml Ethanol gelöst, mit 60 ml 2 N NaOH versetzt und 4 Stunden bei 50°C gerührt. Anschließend wurde das Ethanol abgedampft, der Rückstand mit Wasser versetzt und dreimal mit Essigester extrahiert. Nach dem Ansäuern mit 2 N HCl wurde die Wasserphase dreimal mit Essigester extrahiert, die organische Phase getrocknet und eingedampft. Der Rückstand wurde über eine Kieselgelsäule im Laufmittel Ligroin/Diethylether/Eisessig (60 : 5 : 0.5) getrennt. Es wurden 13.2 g (Ausbeute : 100% d.Th.) der Titelverbindung mit dem Schmelzpunkt 143-144°C erhalten.

Durch Verseifung der entsprechenden Ester wurden auf analoge Weise erhalten :

1) 4-[3-(2-Chlorphenylsulfenyl)-propan-1-yl]-phenylessigsäure
Ausbeute : 66% d.Th. ; Fp : 99-101°C (Ligroin).

2) 4-[3-(2-Chlorphenylsulfonyl)-propan-1-yl]-phenylessigsäure
Ausbeute : 96% d.Th. ; Fp : 154-155°C.

3) 4-[3-(2-Methylphenylsulfinyl)-propan-1-yl]-phenylessigsäure
Ausbeute : 95% d.Th. ; Fp : 108-110°C (Diethylether).

4) 4-[3-(3-Chlorphenylsulfinyl)-propan-1-yl]-phenylessigsäure
Ausbeute : 100% d.Th. ; Fp : 93-94°C (Ligroin/Diethylether).

5) 4-[3-(3-Chlorphenylsulfonyl)-propan-1-yl]-phenylessigsäure
Ausbeute : 98% d.Th. ; Fp : 150-152°C (Ligroin/Diethylether).

6) 4-[3-(3-Trifluormethylphenylsulfonyl)-propan-1-yl]-phenylessigsäure
Ausbeute : 60% d.Th. ; Fp : 170-172°C (Ligroin).

7) 4-[4-(2-Chlorphenylsulfinyl)-butan-1-yl]-phenylessigsäure
Ausbeute : 96% d.Th. ; Öl.

8) 4-[4-(2-Chlorphenylsulfonyl)-butan-1-yl]-phenylessigsäure
Ausbeute : 92% d.Th. ; Öl.

9) 4-[4-(3-bromphenylsulfinyl)-butan-1-yl]-phenylessigsäure
Ausbeute : 96% d.Th. ; Öl.

10) 4-[4-(3-Bromphenylsulfonyl)-butan-1-yl]-phenylessigsäure
Ausbeute : 97% d.Th. ; Fp : 100-105°C (Diethylether).

11) 4-[4-(2-Methylphenylsulfenyl)-butan-1-yl]-phenylessigsäure
Ausbeute : 88% d.Th. ; Öl.

12) 4-[4-(2-Methylphenylsulfinyl)-butan-1-yl]-phenylessigsäure
Ausbeute : 100% d.Th. ; Öl.

13) 4-[4-(2-Methylphenylsulfonyl)-butan-1-yl]-phenylessigsäure
Ausbeute : 98% d.Th. ; Öl.

14) 4-[4-(3-Methylphenylsulfinyl)-butan-1-yl]-phenylessigsäure
Ausbeute : 100% d.Th. ; Öl.

15) 4-[4-(4-Cyanophenylsulfonyl)-butan-1-yl]-phenylessigsäure
Ausbeute : 92% d.Th. ; Fp : 119-120°C (Ethanol/$H_2O$).

16) 4-[4-(4-Methoxyphenylsulfenyl)-butan-1-yl]-phenylessigsäure
Ausbeute : 75% d.Th. ; Fp : 55-70°C (Ethanol).

17) 4-<4-[3-(4-Chlorphenylsulfonyl)-propan-1-yl]-phenyl>-4-oxo-butansäure
Ausbeute : 90% d.Th. ; Fp : 154-157°C.

18) 4-<4-[4-(4-Chlorphenylsulfonyl)-butan-1-yl]-phenyl>-4-oxo-butansäure
Ausbeute : 90% d.Th. ; Öl.

19) 6-<4-[3-(4-Chlorphenylsulfenyl)-propan-1-yl]-phenyl>-hexansäure
Ausbeute : 83% d.Th. ; Fp : 80-81°C (Ethanol).

20) 6-<4-[3-(4-Chlorphenylsulfonyl)-propan-1-yl]-phenyl>-hexansäure
Ausbeute : 75% d.Th. ; Fp : 135-136°C (Essigsäure).

Beispiel 7

4-[3-(4-Chlor-phenylsulfenyl)-propan-1-yl]-acetophenon

a) 3-(4-Chlor-phenylsulfenyl)-propylbenzol

Zu einer 0.4 g-Atom Na enthaltenden 30proz. Natrium-methylatlösung gibt man unter Rühren 57.8 g (0.4 mol) 4-Chlor-thiophenol, dann tropft man innerhalb einer Std. 79.6 g (0.4 mol) 1-Brom-3-phenyl-propan zu und erhitzt eine Std. auf Rückflußtemperatur. Nach dem Abkühlen wird in 1.5 L Wasser eingerührt, mit Ether ausgeschüttelt und die Etherphase getrocknet ($Na_2SO_4$). Nach dem Abdampfen des Ethers wird der Rückstand i. Vakuum destilliert. Ausb. 88.6 g (84% d.Th.), Sdp. 158°C/0.13 mbar. $n_D$ = 1.6070.

b) 4-[3-(4-Chlor-phenylsulfenyl)-propan-1-yl]-acetophenon

Zu 88.0 g (335 mmol) 3-(4-Chlor-phenylsulfenyl)-propylbenzol in 600 ml Methylenchlorid gibt man 28.9 g (369 mmol) Acetylchlorid, kühlt und gibt bei einer Temperatur von 5-10°C portionsweise 133.3 g (1.0 mol) Aluminiumchlorid unter Rühren hinzu. Danach wird eine Std. bei 5-10°C und drei Stdn. bei Raumtemperatur nachgerührt. Man gießt nun in ein Eis-Salzsäure-Gemisch, trennt die Methylenchloridphase ab und wäscht sie mit Wasser, trocknet ($Na_2SO_4$) und dampft sie ein. Das Rohprodukt wird aus einem Heptan-Toluol-Gemisch umkristallisiert. Ausb. 74.0 g (73% d.Th.), Schmp. 42-45°C.

In zu b) analoger Weise erhält man durch Acylieren mittels Acetylchlorid
2) aus Phenyl-phenethyl-sulfon :
4-[2-Phenylsulfonyl)-ethyl]-acetophenon
Ausbeute : 67% d.Th. ; Fp : 100°C (Heptan + Toluol).
3) aus 4-Chlorphenyl-phenethyl-sulfon :
4-[2-(4-Chlor-phenylsulfonyl)-ethyl]-acetophenon
Ausbeute : 76% d.Th. ; Fp : 114-115°C (Heptan).
und durch Acylieren mittels Propionylchlorid
4) aus 4-Chlorphenyl-phenethyl-sulfon :
4-[2-(4-Chlor-phenylsulfonyl)-ethyl]-propiophenon
Ausbeute : 66% d.Th. ; Fp : 99°C (Heptan + Toluol).
Durch analoge Friedel-Crafts-Acylierung mit Bernsteinsäure-ethylester-chlorid erhält man
aus 4-Chlorphenyl-phenylpropyl-sulfon :
5) 4-<4-[3-(4-Chlor-phenylsulfonyl)-propan-1-yl]-phenyl>-4-oxo-butansäure-ethylester
Ausbeute : 81% d.Th. ; Fp : 85°C (Ethanol).

Beispiel 8

1-[2-(Benzolsulfonyl)-ethyl]-4-(1-hydroxyethyl)-benzol

Zu einer Lösung aus 10.0 g (35 mmol) 4-[2-(Benzolsulfonyl)-ethyl]-acetophenon, 100 ml Ethanol und 37.5 ml Methanol gibt man portionsweise insgesamt 1.3 g (35 mmol) Natriumborhydrid, rührt anschließend 1.5 Stdn. und engt dann im Vak. ein. Der Rückstand wird in Eiswasser eingerührt, dann extrahiert man mit Methylenchlorid. Nach Trocknen des Extraktes mit $Na_2SO_4$ dampft man ein. Zum Rückstand gibt man Heptan und setzt nun so lange heißes Toluol zu, bis eben Lösen eingetreten ist. Nach starkem Abkühlen gebildetes Kristallisat wird abgesaugt. Ausbeute : 7.5 g (74% d.Th.) ; Schmp. 64-65°C.

In analoger Weise stellt man aus den entsprechenden Acetophenonen dar :
2) 1-[2-(4-Chlor-phenylsulfonyl)-ethyl]-4-(1-hydroxyethyl)-benzol
Ausbeute : 76% d.Th. ; Schmp. 99-100°C (Heptan + Toluol).
In analoger Weise erhält man aus dem entsprechenden Propiophenon :
3) 1-[2-(4-Chlor-phenylsulfonyl)-ethyl]-4-(1-hydroxypropyl)-benzol
Ausbeute : 74% d.Th. ; Schmp. 91-92°C (Heptan + Toluol).

Beispiel 9

4-[3-(4-Chlor-phenylsulfonyl)-propan-1-yl]-phenethylalkohol

Zu 5.5 ml eiskaltem abs. Ether gibt man unter Rühren portionsweise 736 mg (5.52 mmol) $AlCl_3$, dann 212

mg (5.52 mmol) $LiAlH_4$. Dann wird bei −12°C Innentemperatur eine Lösung aus 1.3 g (3.68 mmol) 4-[3-(4-Chlor-phenylsulfonyl)-propan-1-yl]-phenylessigsäure-ethylester und 10 ml abs. THF zugetropft und eine Std. bei −12°C nachgerührt. Man zersetzt dann durch tropfenweise Zugabe von verd. Natronlauge, saugt ab, wäscht den Filterkuchen mit Methylenchlorid und dampft die vereinigten Filtrate ein. Der Rückstand wird in Methylenchlorid aufgenommen. Man wäscht die Methylenchloridphase mit Wasser, trocknet mit $Na_2SO_4$ und dampft ein. Nach Umkristallisieren aus Ethanol 0.7 g (86% d.Th.) Produkt mit dem Schmp. 105-106°C.

Durch Reduktion mit Lithiumalanat wurde aus dem entsprechenden Phenylessigsäure-ethylester die folgende Verbindung dargestellt :

2) 4-[2-(Benzolsulfonyl)-ethyl]-phenethylalkohol

Eine Lösung aus 5.3 g (16 mmol) 4-[2-(Benzolsulfonyl)-ethyl]-phenylessigsäure-ethylester und 50 ml Ether wird zu einer Suspension aus 0.5 g (12 mmol) Lithiumalanat und 50 ml Ether so zugetropft, daß der Ansatz leicht siedet. Dann läßt man eine Std. nachreagieren und arbeitet auf wie oben beschrieben.
Ausbeute : 3.8 g (82% d.Th.) ; Fp : 57-58°C (Ethanol)

Beispiel 10

γ-Lacton der 4-<4-[3-(4-Chlor-phenylsulfonyl)-propan-1-yl]phenyl>-4-hydroxy-butansäure

Zu einem Gemisch aus 8.16 g (20 mmol) 4-<4-[3-(4-Chlorphenylsulfonyl)-propan-1-yl]-phenyl>-4-oxo-butansäuremethylester und 200 ml Isopropanol gibt man portionsweise insgesamt 0.30 g Natriumborhydrid und rührt anschließend 48 Stdn. mit 10 ml 1 N Salzsäure, rührt eine weitere Stunde und gibt dann 50 ml 2 N Natronlauge zu. Man erwärmt auf 50°C und destilliert nach 4 Stdn. das Lösungsmittel ab. Der Rückstand wird in Diethylether aufgenommen und dreimal gegen Wasser extrahiert. Die wäßrige Phase wird mit 2 N Salzsäure angesäuert, dreimal mit Essigester ausgeschüttelt, die vereinigten organischen Phasen nochmal mit Wasser gewaschen, über Natriumsulfat getrocknet und eingedampft. Man erhält 6.7 g eines festen Rückstandes, der aus Essigester umkristallisiert wird.
Ausbeute : 4.2 g (52% d.Th.) ; Fp : 133-134°C (Zers.)

Beispiel 11

1-<4[3-(4-Chlorphenylsulfonyl)-propan-1-yl]-phenyl>-butandiol-1.4

Zu einem Gemisch aus 1.52 g (40 mmol) Lithiumalanat und 100 ml wasserfreiem THF tropft man langsam eine Lösung aus 4-<4-[3-(4-Chlor-phenylsulfonyl)-propan-1-yl]-phenyl>-4-oxo-butansäure-methylester und 50 ml abs. THF, rührt weitere zwei Stdn. und zersetzt dann unter Kühlen mit Essigester. Nach Zugabe von 1.5 ml 10proz. Natronlauge wird filtriert und das Filtrat eingedampft. Der ölige Rückstand wird an Kieselgel mit einem Gemisch aus Ligroin + Essigester + Eisessig (1.5 + 1 + 0.0065 Vol.), dann mit Essigester chromatographiert. Nach Abdestillieren des Lösungsmittels wird mit Ether plus Ligroin zur Kristallisation gebracht und abgesaugt.
Ausbeute : 2.0 g (30% d.Th.) ; Fp : 90-97°C (Zers.).

Beispiel 12

4-[3-(4-Chlor-phenethylsulfenyl)-propan-1-yl]-phenoxyessigsäure-methylester

a) 4-(3-Hydroxypropyl)-phenoxyessigsäure-ethylester

Man erhitzt ein Gemisch aus 10.0 g (66 mmol) 4-(3-Hydroxypropyl)-phenol, 13.7 g (99 mmol) pulv. $K_2CO_3$ sicc. und 100 ml Butanon eine Std. lang auf 80°C, gibt 13.2 g (79 mmol) Bromessigsäure-ethylester und einige Kristalle Kaliumiodid zu und hält weitere 5 Stdn. bei 80°C. Dann wird abgesaugt, mit Aceton nachgewaschen und eingedampft. Das zurückbleibende Öl (Ausb. quant.) wird roh weiterverarbeitet.

b) 4-(3-Brompropyl)-phenoxyessigsäure-ethylester

Zu einem Gemisch aus 5.75 g (21.3 mmol) $PBr_3$ und 60 ml Ether tropft man eine Lösung aus 15.2 g (63.8 mmol) 4-(3-Hydroxypropyl)-phenoxyessigsäure-ethylester und hält anschließend drei Stdn. auf Rückflußtemperatur. Dann wird im Vak. der Ether abdestilliert, der Rückstand in Essigester aufgenommen und die Lösung mit gesätt. $NaHCO_3$-Lösung, dann zweimal mit Wasser gewaschen. Nach Trocknen mit $Na_2SO_4$ dampft man ein und reinigt durch Säulenchromatographie (Kieselgel/Methylenchlorid). Ausbeute 12.9 g (67% d.Th.) ; farbloses Öl.

c) 4-[3-(4-Chlor-phenethylsulfenyl)-propan-1-yl]-phenoxyessigsäure-methylester

Zu 6.4 ml einer 30proz. Lösung von Natriummethylat in Methanol (entspr. 34.5 mmol NaOMe) tropft man 5.68 g (32.9 mmol) 4-Chlor-phenethylmercaptan, dann eine Lösung aus 9.9 g (32.9 mmol) 4-(3-Brompropyl)-phenoxyessigsäure-ethylester, und hält eine Std. auf Rückflußtemperatur. Dann wird in 300 ml Eiswasser eingerührt, mit Ether extrahiert und die Lösung mit $Na_2SO_4$ getrocknet. Nach Eindampfen des Ethers chromatographiert man an Kieselgel, zunächst mit n-Heptan + Methylenchlorid (9 + 1 Vol.), dann mit reinem Methylenchlorid. Ausbeute : 10.6 g (85% d.Th.) ; farbloses Öl.

In zu c) analoger Weise wurden enthalten :

— aus 2-(2-Bromethyl)-phenoxyessigsäure-methylester und 4-Chlor-thiophenol :

2) 2-[2-(4-Chlor-phenylsulfenyl)-ethyl]-phenoxyessigsäure-methylester

Ausbeute : 72% d.Th. ; Fp : 50°C (Ligroin).

— aus 2-(3-Brompropyl)-phenoxyessigsäure-ethylester und 4-Chlor-thiophenol :

3) 2-[3-(4-Chlor-phenylsulfenyl)-propan-1-yl]-phenoxyessigsäure-ethylester

Ausbeute : 70% d.Th. ; Öl.

— aus 3-(3-Brompropyl)-phenoxyessigsäure-ethylester und Thiophenol :

4) 3-[3-(Phenylsulfenyl)-propan-1-yl]-phenoxyessigsäure-ethylester

Ausbeute : 90% d.Th. ; farbloses Öl.

und 4-Brom-thiophenol :

5) 3-[3-(4-Brom-phenylsulfenyl)-propan-1-yl]-phenoxyessigsäure-ethylester

Ausbeute : 72% d.Th. ; farbloses Öl.

und 4-Chlor-thiophenol :

6) 3-[3-(4-Chlor-phenylsulfenyl)-propan-1-yl]-phenoxyessigsäure-ethylester

Ausbeute : 75% d.Th. ; Öl.

und aus 4-(3-Brompropyl)phenoxyessigsäure-ethylester und n-Hexylmercaptan :

7) 4-[3-(n-Hexylsulfenyl)-propan-1-yl]-phenoxyessigsäure-ethylester

Ausbeute : 83% d.Th. ; farbloses Öl.

und 4-Chlor-thiophenol :

8) 4-[3-(4-Chlorphenylsulfenyl)-propan-1-yl]-phenoxyessigsäure-ethylester

Ausbeute : 69% d.Th. ; Öl.

und 4-Phenyl-thiophenol :

9) 4-[3-(4-Phenyl-phenylsulfenyl)-propan-1-yl]-phenoxyessigsäure-ethylester

Ausbeute : 68% d.Th. ; Schmp. 118-120°C.

Beispiel 13

4-[4-(4-Chlor-phenylsulfenyl)-butan-1-yl]-phenyoxyessigsäureethylester

a) 4-(4-Methoxyphenyl)-4-oxo-buttersäure-methylester

Man löst 32.66 g (302 mmol) Anisol und 50.0 g (322 mmol) Bernsteinsäuremethylesterchlorid in 500 ml Methylenchlorid und gibt bei 5 bis 10°C portionsweise 120.8 g (903 mmol) $AlCl_3$ zu. Man rührt eine Std. bei Eisbadtemperatur, dann 3 Stdn. bei Raumtemperatur nach, gießt auf Eis-HCl-Gemisch und trennt die org. Phase ab. Sie wird mit Wasser gewaschen, mit verd. NaOH extrahiert, dann wäscht man erneut mit Wasser und trocknet mittels $Na_2SO_4$. Nach Eindampfen wird der ölige Rückstand destilliert. Ausb. 34.8 g (52% d.Th.) farblose, bei 49-50°C schmelzende Substanz mit dem Sdp. 142-144°C/0.13 mbar.

b) 4-(4-Methoxyphenyl)-buttersäure-methylester

Man hydriert 30.5 g (137 mmol) des nach a) erhaltenen Ketoesters in einem Gemisch aus 300 ml Methanol und 10 ml HCl-enthaltendem Ether unter Zusatz von Palladiumkohle bei 40 mbar und erhält in quantitativer Ausbeute den Phenylbuttersäuremethylester als farbloses Öl.

c) 4-(4-Hydroxybutyl)-anisol

erhält man durch Reduktion des 4-(4-Methoxyphenyl)-buttersäuremethylesters mittels $LiAlH_4$ in etheri-

scher Lösung in praktisch quantitativer Ausbeute als farbloses Öl.

d) 4-(4-Brombutyl)-phenol

Man hält ein Gemisch aus 19.0 g (105 mmol) 4-(4-Hydroxybutyl)-anisol, 60 ml 48proz. HBr und 35 ml Eisessig 4 Stdn. auf Rückflußtemperatur, kühlt ab, rührt in Eiswasser ein und ethert aus. Die Etherphase wird dreimal mit verd. NaOH extrahiert. Durch Zugabe von Salzsäure fällt man aus der Natronlaugephase das freie Phenol aus. Man extrahiert das Phenol mit Ether, wäscht die Etherphase mit Wasser, trocknet ($Na_2SO_4$) und dampft den Ether ab. Der ölige Rückstand wird an einer Kieselgel-Säule mit Toluol als Laufmittel gereinigt. Ausb. 13.0 g (54% d.Th.) ; farbloses Öl.

e) Tetrahydropyranylether des 4-(4-Brombutyl)-phenols

Zu 28.6 g (0.34 mol) Dihydropyran tropft man bei Raumtemperatur ein Gemisch aus 39.0 g (0.17 mol) 4-(4-Brombutyl)-phenol und 100 ml Methylenchlorid, rührt zwei Stdn. nach und destilliert nun i.Vak. Methylenchlorid und überschüss. Dihydropyran ab. Als Rückstand bleibt in quant. Ausbeute ein öliges Produkt, das roh in die nächste Stufe eingesetzt wird.

f) Tetrahydropyranylether des 4-[4-(4-Chlor-phenylsulfenyl)-butan-1-yl]-phenols

Zu einem Gemisch aus 23.1 g (0.16 mol) 4-Chlorthiophenol und 0.16 mol Natriummethylat (in Form einer 30proz. methanol. Lösung) tropft man langsam eine Lösung aus 50.0 g (0.16 mol) des nache) erhaltenen Tetrahydropyranylethers und 20 ml Methanol, erhitzt anschließend drei Stdn. auf Rückflußtemperatur, kühlt ab und gießt in 1 L Wasser. Nun wird mit Ether extrahiert und die Etherphase mit $Na_2SO_4$ getrocknet und eingedampft. Nach Reinigen über Kieselgel/Methylenchlorid plus Ligroin (1 : 1 Vol.) erhält man 42.3 g (70% d.Th.) Produkt als farbloses Öl.

g) 4-[4-(4-Chlor-phenylsulfenyl)-butan-1-yl]-phenol

erhält man in quant. Rohausbeute, indem man den nach f) erhaltenen Tetrahydropyranylether in einem Gemisch aus Aceton und 2N-HCl rührt, Aceton abdestilliert, den Rückstand mit Wasser verdünnt, ausethert und die Etherphase nach Waschen mit Wasser trocknet ($Na_2SO_4$) und eindampft. Farbloses Öl.

h) 4-[4-(4-Chlor-phenylsulfenyl)-butan-1-yl]-phenoxyessigsäure-ethylester

Man rührt ein Gemisch aus 34.8 g (120 mmol) 4-[4-(4-Chlorphenylsulfenyl)-butan-1-yl]-phenol, 400 ml Butanon und 18.1 g (130 mmol) pulverisiertem, trocknem Kaliumcarbonat eine Std. lang bei 90°C, kühlt etwas ab, gibt 15.9 g (130 mmol) Chloressigsäure-ethylester zu und hält 15 Stdn. auf Rückflußtemperatur. Durch Zugabe weiterer 7.95 g (65 mmol) Chloressigsäureethylester und Erhitzen bringt man die Reaktion zu Ende, saugt noch heiß ab, wäscht den Filterkuchen mit warmem Aceton und dampft die vereinigten organischen Phasen ein. Nach Reinigen über Kieselgel/Ligroin + Toluol (10 : 1 Vol.) erhält man 26.4. g (59% d.Th.) farbloses öliges Produkt.

Beispiel 14

Durch Oxidation von 4-[3-(4-Chlor-phenylsulfenyl)-propan-1-yl]-phenoxyessigsäure mittels $H_2O_2$/Essigsäure in Analogie zu Beispiel 3 erhält man
4-[3-(4-Chlor-phenylsulfinyl)-propan-1-yl]-phenoxyessigsäure
Ausbeute : 55% d.Th. ; Fp : 126-127°C (Ethylacetat).

Beispiel 15

4-[4-(4-Chlorphenylsulfinyl)-butan-1-yl]-phenoxyessigsäureethylester

Ein Gemisch aus 7.0 g (18.4 mmol) 4-[4-(4-Chlorphenylsulfenyl)-butan-1-yl]-phenoxyessigsäureethylester, 4.34 g (20.3 mmol) $NaIO_4$, 210 ml Wasser und 250 ml Methanol wurden 5 Stunden bei Raumtemperatur, dann 5 Stunden bei 50°C gerührt. Anschließend destilliert man im Vakuum das Methanol ab, gibt zum Rückstand Wasser und ethert aus. Die Etherphase wird mit $Na_2SO_4$ getrocknet und eingedampft. Nach Reinigung über

eine Kieselgelsäule im Laufmittel $CH_2Cl_2$/Methanol (100 : 1) wurden 6.4 g (87% d.Th.) der Titelverbindung mit dem Schmelzpunkt 50-51°C erhalten.

Beispiel 16

4-[4-(4-Chlorphenylsulfonyl)-butan-1-yl]-phenoxyessigsäureethylester

Man läßt ein Gemisch aus 3.95 g (10 mmol) 4-[4-(4-Chlorphenylsulfinyl)-butan-1-yl]-phenoxyessigsäure-ethylester, 35 ml Eisessig und 2.8 ml 30% $H_2O_2$ 5 Tage bei Raumtemperatur stehen. Dann verdünnt man mit Wasser, extrahiert mit Methylenchlorid und wäscht die organische Phase mehrmals mit Wasser. Nach Trocknen mit $Na_2SO_4$ wird eingedampft und das Produkt über eine Kieselgelsäule in Methylenchlorid gereinigt. Man erhält 2.75 g (67% d.Th.) der Titelverbindung mit dem Schmelzpunkt 63-64°C.

Beispiel 17

In einer zu Beispiel 7 analogen Verfahrensweise wurden aus den entsprechenden Sulfenyl-phenoxyessigsäureestern folgende Sulfonyl-phenoxyessigsäureester hergestellt:

1) 2-[2-(4-Chlor-phenylsulfonyl)-ethyl/-phenoxyessigsäure-methylester
Ausbeute : 83% d.Th. ; Fp : 87-88°C (Methanol).
2) 2-[3-(4-Chlorphenylsulfonyl)-propan-1-yl]-phenoxyessigsäureethylester
Ausbeute : 60% d.Th. ; Öl.
3) 3-[3-(Phenylsulfonyl)-propan-1-yl]-phenoxyessigsäureethylester
Ausbeute : 59% d.Th. ; farbloses Öl.
4) 3-[3-(4-Brom-phenylsulfonyl)-propan-1-yl]-phenoxyessigsäure-ethylester
Ausbeute : 71% d.Th. ; farbloses Öl.
5) 4-[3-n-Hexyl-sulfonyl)-propan-1-yl]-phenoxyessigsäure-ethyleter
Ausbeute : 75% d.Th. ; Öl, teilweise kristallisierend.
6) 4-[3-(4-Chlor-phenethylsulfonyl)-propan-1-yl]-phenoxyessigsäure-methylester
Ausbeute : 85% d.Th. ; Fp : 92-93°C (66proz. Ethanol).
7) 4-[3-(4-Phenyl-phenylsulfonyl)-propan-1-yl]-phenoxyessigsäure-ethylester
Ausbeute : 91% d.Th. ; Fp : 90-94°C (Ether + Ligroin).

Beispiel 18

3-[3-(4-Chlor-phenylsulfonyl)-propan-1-yl]-phenoxyessigsäure-ethylester

Man hält ein Gemisch aus 4.0 g (11 mmol) 3-[3-(4-Chlor-phenylsulfenyl)-propan-1-yl]-phenoxyessigsäure-ethylester, 35 ml Ethanol, 15 ml Wasser und 4.3 g (22 mmol) Natriumperiodat zwei Stdn. bei 75°C, gibt nochmals 2.0 g Periodat zu und rührt weitere drei Stdn. bei 75°C. Danach wird eingedampft, mit Wasser versetzt und mit Ether extrahiert. Man trocknet den Etherextrakt mit $Na_2SO_4$ und dampft ein. Ausbeute : quantitativ ; farbloses Öl.

Beispiel 19

Durch Verseifen der entsprechenden Phenoxyessigsäureester in einer zu Beispiel 6 analogen Verfahrensweise werden die nachstehend aufgeführten Phenoxyessigsäuren dargestellt:

1) 2-[2-(4-Chlor-phenylsulfenyl)-ethyl]-phenoxyessigsäure
Ausbeute : 87% d.Th. ; Fp : 115°C (Essigsäure).
2) 2-[2-(4-Chlor-phenylsulfonyl)-ethyl]-phenoxyessigsäure
Ausbeute 83% d.Th. ; Fp : 201-202°C (Essigsäure).
3) 2-[3-(4-Chlor-phenylsulfonyl)-propan-1-yl]-phenoxyessigsäure
Ausbeute : 94% d.Th. ; Fp : 87-89°C (Ligroin).
4) 3-[3-(Phenylsulfonyl)-propan-1-yl]-phenoxyessigsäure
Ausbeute : 93% d.Th ; farbloses Öl.
5) 3-[3-(4-Chlor-phenylsulfonyl)-propan-1-yl]-phenoxyessigsäure
Ausbeute : 78% d.Th. ; Fp : 123-124°C (Toluol).
6) 3-[3-(4-Brom-phenylsulfonyl)-propan-1-yl]-phenoxyessigsäure
Ausbeute : 45% d.Th. ; Fp : 135-135.5°C (Essigester).

7) 4-[3-(Methansulfonyl)-propan-1-yl]-phenoxyessigsäure
Ausbeute : 87% d.Th. ; Fp : 139-140°C.
8) 4-[3-(n-Hexansulfonyl)-propan-1-yl]-phenoxyessigsäure
Ausbeute : 63% d.Th. ; Fp : 133-135°C (Ligroin).
9) 4-[3-(4-Chlorphenethylsulfenyl)-propan-1-yl]-phenoxyessigsäure
Ausbeute : 80% d.Th. ; Fp : 91-92°C (66proz. Ethanol).
10) 4-[3-(4-Chlor-phenethylsulfonyl)-propan-1-yl]-phenoxyessigsäure
Ausbeute : 94% d.Th. ; Fp : 159-160°C (Ethanol).
11) 4-[3-(4-Chlorphenylsulfenyl)-propan-1-yl]-phenoxyessigsäure
Ausbeute : 95% d.Th. ; Fp : 123-124°C (i-Hexan).
12) 4-[3-(4-Chlor-phenylsulfonyl)-propan-1-yl]-phenoxyessigsäure
Ausbeute : 52% d.Th. ; Fp : 164°C (Ethylacetat).
13) 4-[3-(4-Phenyl-phenylsulfonyl)-propan-1-yl]-phenoxyessigsäure
Ausbeute : 92% d.Th. ; Fp : 144-147°C (Eisessig + Wasser).
14) 4-[4-(4-Chlorphenylsulfenyl)-butan-1-yl]-phenoxyessigsäure
Ausbeute : 93% d.Th. ; Fp : 135-136°C ($H_2O$, Ethanol).
15) 4-[4-(4-Chlorphenylsulfinyl)-butan-1-yl]-phenoxyessigsäure
Ausbeute : 87% d.Th. ; Fp : 106-107°C (wäßr. Ethanol).
16) 4-[4-(4-Chlorphenylsulfonyl)-butan-1-yl]-phenoxyessigsäure
Ausbeute : 93% d.Th. ; Fp : 106-107°C ($H_2O$, Ethanol).
17) 3-[4-(4-Chlorphenylsulfonyl)-butan-1-yl]-phenoxyessigsäure
Ausbeute : 81% d.Th. ; Fp : 115°C ($H_2O$, Ethanol).
18) 4-[2-(Phenylsulfonyl)-ethyl]-phenoxyessigsäure
Ausbeute : 95% d.Th. ; Fp : 179-181°C (Ethanol).
19) 4-[3-(Phenylsulfonyl)-propan-1-yl]-phenoxyessigsäure
Ausbeute : 71% d.Th. ; Fp : 51-52°C (Ethanol).

## Patentansprüche

1. Verbindungen der Formel I

$$R_1-\overset{(O)_m}{\overset{|}{S}}-B-W-A \qquad (I).$$

in welcher

- $R_1$ eine $C_1$-$C_6$-Alkyl- oder $C_2$-$C_6$-Alkenylgruppe, einen $C_3$-$C_7$-Cycloalkylrest, einen Aralkyl-, Aralkenyl- oder Arylrest, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_{16}$ Acyl oder Azid substituiert sein kann, und der Alkyl- oder Alkenylteil 1-5 bzw. 2-3 C-Atome aufweisen kann,
- m die Zahlen 0, 1 oder 2,
- B eine unverzweigte oder verzweigte, gesättigte Alkylenkette mit maximal 6 C-Atomen,
- W 1.2-, 1.3- oder 1.4-Phenylen,
- A einen Oxyessigsäurerest bedeuten,

sowie deren pharmakologisch verträglichen Salze, Ester und Amide.

2. Verbindungen der Formel I

$$R_1-\overset{(O)_m}{\overset{|}{S}}-B-W-A \qquad (I).$$

in welcher

$R_1$ einen Aralkyl-, Aralkenyl- oder Arylrest, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_{16}$ Acyl oder Azid substituiert sein kann, und der Alkyl- oder Alkenylteil 1-5 bzw. 2-3 C-Atome aufweisen kann,

m die Zahlen 0, 1 oder 2,

B eine unverzweigte oder verzweigte, gesättigte Alkylenkette mit maximal 6 C-Atomen,

W 1.2-, 1.3- oder 1.4-Phenylen,

A eine Formyl-$C_1$-$C_5$-alkylgruppe, eine Hydroxy-$C_0$-$C_6$-alkylgruppe, einen Oxyessigsäurerest oder einen Rest D-$R_2$ darstellt, in dem D eine

$$-\underset{O}{\overset{\|}{C}}- \text{ oder } -\underset{OH}{\overset{|}{CH}}\text{-Gruppe}$$

und $R_2$ Wasser Wasserstoff, einen $C_1$-$C_5$ Alkyl-, einen Hydroxy-$C_1$-$C_5$-alkyl- oder einen Carboxy-$C_0$-$C_4$-alkylrest und gegebenenfalls deren Lactone bedeuten,
sowie deren pharmakologisch verträglichen Salze, Ester und Amide.

3. Verbindungen der Formel I gemäß Anspruch 1 oder 2, betreffend
3-[3-(Phenylsulfonyl)-propan-1-yl]-phenoxyessigsäure ;
3-[3-(4-Chlorphenylsulfonyl)-propan-1-yl-phenoxyessigsäure ;
3-[3-(4-Bromphenylsulfonyl)-propan-1-yl]-phenoxyessigsäure ;
4-[3-(4-Chlorphenylsulfonyl)-propan-1-yl]-phenoxyessigsäure
oder
3-[4-(4-Chlorphenylsulfonyl)-butan-1-yl]-phenoxyessigsäure.

4. Die Verbindungen
4-[3-(2-Methylphenylsulfonyl)-propan-1-yl]-phenylessigsäure
4-[3-(2-Chlorphenylsulfenyl)-propan-1-yl]-phenylessigsäure
4-[3-(2-Chlorphenylsulfonyl)-propan-1-yl]-phenylessigsäure
4-[3-(2-Methylphenylsulfinyl)-propan-1-yl]-phenylessigsäure
4-[3-(3-Chlorphenylsulfinyl)-propan-1-yl]-phenylessigsäure
4-[3-(3-Chlorphenylsulfonyl)-propan-1-yl]-phenylessigsäure
4-[3-(3-Trifluormethylphenylsulfonyl)-propan-1-yl]-phenylessigsäure
4-[4-(2-Chlorphenylsulfinyl)-butan-1-yl]-phenylessigsäure
4-[4-(2-Chlorphenylsulfonyl)-butan-1-yl]-phenylessigsäure
4-[4-(3-Bromphenylsulfinyl)-butan-1-yl]-phenylessigsäure
4-[4-(3-Bromphenylsulfonyl)-butan-1-yl]-phenylessigsäure
4-[4-(2-Methylphenylsulfenyl)-butan-1-yl]-phenylessigsäure
4-[4-(2-Methylphenylsulfinyl)-butan-1-yl]-phenylessigsäure
4-[4-(2-Methylphenylsulfonyl)-butan-1-yl]-phenylessigsäure
4-[4-(3-Methylphenylsulfinyl)-butan-1-yl]-phenylessigsäure
4-[4-(4-Cyanophenylsulfonyl)-butan-1-yl]-phenylessigsäure
4-[4-(4-Methoxyphenylsulfenyl)-butan-1-yl]-phenylessigsäure
6-<4-[3-(4-Chlorphenylsulfenyl)-propan-1-yl]-phenyl>-hexansäure
oder
6-<4-[3-(4-Chlorphenylsulfonyl)-propan-1-yl]-phenyl>-hexansäure.

5. Verfahren zur Herstellung von Oxyessigsäuren gemäß Anspruch 1, 2 oder 3, dadurch gekennzeichnet, daß man eine Verbindung der Formel IX

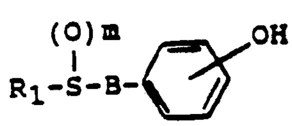

(IX),

in der $R_1$, B und m die oben angeführte Bedeutung haben, mit Chlor- oder Bromessigsäure bzw. deren Estern umsetzt, und anschließend gegebenenfalls die erhaltenen Verbindungen der Formel I in andere Verbindungen der Fomel I in an sich bekannter Weise umwandelt, oder die erhaltenen freien Säuren in deren pharmakologisch verträgliche Salze, Ester oder Amide bzw. die erhaltenen Ester oder Amide in die freien Säuren überführt.

6. Verfahren zur Herstellung von Verbindungen gemäß Anspruch 1, 2, 3 oder 4, dadurch gekennzeichnet, daß man in an sich bekannter Weise eine Verbindung der Formel II

X-B-W-A (II),

in welcher B, W und A die oben angegebene Bedeutung haben und X einen reaktiven Rest darstellt, entweder mit einem Mercaptan der Formel III

$R_1$-SH (III),

oder mit einer Sulfinsäure der Formel VI oder eines ihrer Salze

$R_1$-SO-OH (VI),

in denen $R_1$ die angegebene Bedeutung hat,
umsetzt, und anschließend gegebenenfalls die erhaltene Sulfenyl-Verbindung zur Sulfinyl- oder Sulfonyl-Verbindung oxidiert oder die erhaltene Sulfonyl-Verbindung zur Sulfinyl- oder Sulfenyl-Verbindung reduziert, oder die erhaltene SulfinylVerbindung zur Sulfenyl-Verbindung reduziert.

7. Verfahren zur Herstellung von Verbindungen gemäß Anspruch 1 oder 2, denen $R_1$ einen Alkyl-, Alkenyl-, Cycloalkyl- oder Aralkylrest darstellt, dadurch gekennzeichnet, daß man ein Mercaptan der Formel IV

HS-B-W-A (IV),

mit einer Verbindung der Formel V

$R_1$-X (V),

umsetzt, wobei A, B, $R_1$, W und X die angegebene Bedeutung haben und anschließend gegebenenfalls die erhaltenen Verbindungen der Formel I in andere Verbindungen der Formel I in an sich bekannter Weise umwandelt, oder die erhaltenen freien Säuren in deren pharmakologisch verträgliche Salze, Ester oder Amide bzw. die erhaltenen Ester oder Amide in die freien Säuren überführt.

8. Arzneimittel enthaltend eine Verbindung gemäß Anspruch 1, 2, 3 oder 4 neben üblichen Träger- und Hilfsstoffen.

9. Verwendung von Verbindungen gemäß Anspruch 1, 2, 3 oder 4 zur Herstellung von Arzneimitteln zur Behandlung von Stoffwechselerkrankungen.

10. Verwendung von Verbindungen gemäß Anspruch 1, 2, 3 oder 4 zur Herstellung von Arzneimitteln zur Behandlung von Durchblutungsstörungen.

## Revendications

1. Composés de formule I

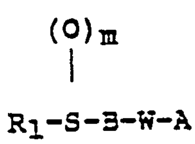

(I),

dans laquelle

$R_1$ représente un groupe alkyle en $C_1$-$C_6$ ou alcényle en $C_2$-$C_6$, un groupe cycloalkyle en $C_3$-$C_7$, un groupe aralkyle, aralcényle ou aryle, le groupe aryle pouvant être substitué une ou plusieurs fois par un halogène, un groupe alkyle en $C_1$-$C_6$, alcoxy en $C_1$-$C_6$, hydroxyle, trifluorométhyle, cyano, nitro, amino, alkylamino en $C_1$-$C_6$, dialkylamino en $C_2$-$C_{12}$, acylamino en $C_1$-$C_6$, acyle en $C_1$-$C_{16}$ ou azide, et le groupe alkyle ou alcényle pouvant comporter 1-5 ou 2-3 atomes de C,

m vaut 0, 1 ou 2,

B représente une chaîne alkylène saturée, droite ou ramifiée, comportant au maximum 6 atomes de C,
W représente un groupe 1.2-, 1.3- ou 1.4-phénylène,
A représente un reste acide oxyacétique,
ainsi que leurs sels, esters et amides pharmaceutiquement acceptables.

2. Composés de formule I

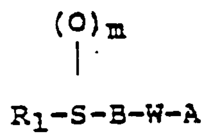

(I),

dans laquelle

$R_1$ représente un groupe aralcényle ou aryle, le groupe aryle pouvant être substitué une ou plusieurs fois par un halogène, un groupe alkyle en $C_1$-$C_6$, alcoxy en $C_1$-$C_6$, hydroxyle, trifluorométhyle, cyano, nitro, amino, alkylamino en $C_1$-$C_6$, dialkylamino en $C_2$-$C_{12}$, acylamino en $C_1$-$C_6$, acyle en $C_1$-$C_{16}$ ou azide, et le groupe alkyle ou alcényle pouvant comporter 1-5 ou 2-3 atomes de C,
m vaut 0, 1 ou 2,
B représente une chaîne alkylène saturée, droite ou ramifiée, comportant au maximum 6 atomes de C,
W représente un groupe 1.2-, 1.3 - ou 1.4-phénylène,
A représente un groupe formylalkyle en $C_1$-$C_5$, un groupe hydroxyalkyle en $C_0$-$C_6$, un groupe acide oxyacétique ou un groupe D-$R_2$, dans lequel D représente un groupe

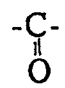

ou

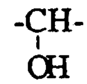

et $R_2$ représente l'hydrogène, un groupe alkyle en $C_1$-$C_5$, un groupe hydroxyalkyle en $C_1$-$C_5$ ou un groupe carboxyalkyle en $C_0$-$C_4$ et éventuellement leur lactone,
ainsi que leurs sels, esters et amides pharmaceutiquement acceptables.

3. Composés de formule I selon la revendication 1 ou 2, qui sont les
acide 3-[3-(phénylsulfonyl)-propane-1-yl]-phénoxyacétique,
acide 3-[3-(4-chlorophénylsulfonyl)-propane-1-yl]-phénoxyacétique,
acide 3-[3-(4-bromophénylsulfonyl)-propane-1-yl]-phénoxyacétique,
acide 4-[3-(4-chlorophénylsulfonyl)-propane-1-yl]-phénoxyacétique,
ou
acide 3-[4-(4-chlorophénylsulfonyl)-butane-1-yl]-phénoxyacétique.

4. Les composés
acide 4-[3-(2-méthylphénylsulfonyl)-propane-1-yl]-phénylacétique
acide 4-[3-(2-chlorophénylsulfényl)-propane-1-yl]-phénylacétique
acide 4-[3-(2-chlorophénylsulfonyl)-propane-1-yl]-phénylacétique
acide 4-[3-(2-méthylphénylsulfinyl)-propane-1-yl]-phénylacétique
acide 4-[3-(3-chlorophénylsulfinyl)-propane-1-yl]-phénylacétique
acide 4-[3-(3-chlorophénylsulfonyl)-propane-1-yl]-phénylacétique
acide 4-[3-(3-trifluorométhylphénylsulfonyl)-propane-1-yl]-phénylacétique
acide 4-[4-(2-chlorophénylsulfinyl)-butane-1-yl]-phénylacétique
acide 4-[4-(2-chlorophénylsulfonyl)-butane-1-yl]-phénylacétique
acide 4-[4-(3-bromophénylsulfinyl)-butane-1-yl]-phénylacétique
acide 4-[4-(3-bromophénylsulfonyl)-butane-1-yl]-phénylacétique
acide 4-[4-(2-méthylphénylsulfényl)-butane-1-yl]-phénylacétique
acide 4-[4-(2-méthylphénylsulfinyl)-butane-1-yl]-phénylacétique
acide 4-[4-(2-méthylphénylsulfonyl)-butane-1-yl]-phénylacétique
acide 4-[4-(3-méthylphénylsulfinyl)-butane-1-yl]-phénylacétique
acide 4-[4-(4-cyanophénylsulfonyl)-butane-1-yl]-phénylacétique
acide 4-[4-(4-métoxyphénylsulfényl)-butane-1-yl]-phénylacétique

acide 6-<4-[3-(4-chlorophénylsulfényl)-propane-1-yl]-phényl>-hexanoïque
ou
acide 6-<4-[3-(4-chlorophénylsulfonyl)-propane-1-yl]-phényl>-hexanoïque.

5. Procédé de préparation des acides oxyacétiques selon la revendication 1, 2 ou 3, caractérisé en ce que l'on fait réagir un composé de formule IV

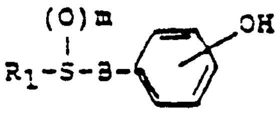

(IX),

dans laquelle $R_1$, B et m ont les significations mentionnées ci-dessus, avec l'acide chloro- ou bromoacétique ou avec leurs esters, et ensuite, on transforme éventuellement les composés de formule I obtenus en d'autres composés de formule I, de manière connue en soi, ou bien on transforme les acides libres obtenus en leurs sels, esters ou amides pharmaceutiquement acceptables, ou l'on transforme les esters ou amides obtenus en acides libres.

6. Procédé de préparation des composés selon les revendications 1, 2, 3 ou 4, caractérisé en ce que, de manière connue en soi, on fait réagir un composé de formule II

X-B-W-A (II),

dans laquelle B, W et A ont les significations mentionnées ci-dessus et X représente un groupe réactif, soit avec un mercaptan de formule III

$R_1$-SH (III),

soit avec un acide sulfinique de formule VI ou un de ses sels

$R_1$-SO-OH (IV),

dans laquelle $R_1$ a la signification mentionnée ci-dessus,
et ensuite, éventuellement, on oxyde le composé sulfényle obtenu en composé sulfinyle ou sulfonyle ou on réduit le composé sulfonyle obtenu en composé sulfinyle ou sulfényle, ou on réduit le composé sulfinyle obtenu en composé sulfényle.

7. Procédé de préparation des composés selon les revendications 1 ou 2, dans lesquels $R_1$ représente un groupe alkyle, alcényle, cycloalkyle ou aralkyle, caractérisé en ce que l'on fait réagir un mercaptan de formule IV

HS-B-W-A (IV),

avec un composé de formule V

$R_1$-X (V),

dans laquelle A, B, $R_1$, W et X ont les significations mentionnées ci-dessus, et ensuite, on transforme éventuellement les composés de formule I obtenus en d'autres composés de formule I, de manière connue en soi, ou on transforme les acides libres obtenus en leurs sels, esters ou amides pharmaceutiquement acceptables, ou l'on transforme les esters ou amides obtenus en acides libres.

8. Médicament contenant un composé selon les revendications 1, 2, 3 ou 4, en plus de véhicules ou adjuvants usuels.

9. Utilisation des composés selons les revendications 1, 2, 3 ou 4, pour la préparation de médicaments pour le traitement de troubles du métabolisme.

10. Utilisation des composés selon les revendications 1, 2, 3 ou 4, pour la préparation de médicaments pour le traitement des troubles de la circulation sanguine.

**Claims**

1. Compounds of the formula I

$$R_1-\overset{\overset{(O)_m}{|}}{S}-B-W-A \qquad (I)$$

in which

$R_1$ signifies a $C_1$-$C_8$-alkyl or $C_2$-$C_8$-alkenyl group, a $C_3$-$C_7$-cycloalkyl radical, an aralkyl, aralkenyl or aryl radical, whereby the aryl moiety can, in each case, be substituted one or more times by halogen, $C_1$-$C_6$-alkyl, $C_1$-$C_6$-alkoxy, hydroxyl, trifluoromethyl, cyano, nitro, amino, $C_1$-$C_6$-alkylamino, $C_2$-$C_{12}$-dialkylamino, $C_1$-$C_6$-acylamino, $C_1$-$C_{16}$-acyl or azido, and the alkyl or alkenyl moiety can have 1-5 and 2-3 C-atoms, respectively,
m the numbers 0, 1 or 2,
B an unbranched or branched, saturated alkylene chain with a maximum of 6 C-atoms,
W 1,2-, 1,3- or 1,4-phenylene,
A an oxyacetic acid radical,
as well as their pharmacologically compatible salts, esters and amides.

2. Compounds of the formula I

$$R_1-\overset{\overset{(O)_m}{|}}{S}-B-W-A \qquad (I),$$

in which

$R_1$ signifies an aralkyl, aralkenyl or aryl radical, whereby the aryl moiety can, in each case, be substituted one or more times by halogen, $C_1$-$C_6$-alkyl, $C_1$-$C_6$-alkoxy, hydroxyl, trifluoromethyl, cyano, nitro, amino, $C_1$-$C_6$-alkylamino, $C_2$-$C_{12}$-dialkylamino, $C_1$-$C_6$-acylamino, $C_1$-$C_{16}$-acyl or azide and the alkyl or alkenyl moiety can have 1-5 and 2-3 C-atoms, respectively,
m the numbers 0, 1 or 2,
B an unbranched or branched, saturated alkylene chain with a maximum of 6 C-atoms,
W 1,2-, 1,3- or 1,4-phenylene,
A a formyl-$C_1$-$C_5$-alkyl group, a hydroxy-$C_0$-$C_6$-alkyl group, an oxyacetic acid radical or a radical D-$R_2$, in which D signifies a

$$-\underset{\underset{O}{\|}}{C}- \quad \text{or} \quad -\underset{\underset{OH}{|}}{CH}-$$

group and $R_2$ hydrogen, a $C_1$-$C_5$-alkyl, a hydroxy-$C_1$-$C_5$-alkyl or a carboxy-$C_0$-$C_4$-alkyl radical and possibly their lactones,
as well as their pharmacologically compatible salts, esters and amides.

3. Compounds of the formula I according to claim 1 or 2, concerning
3-[3-(phenylsulphonyl)-propan-1-yl]-phenoxyacetic acid ;
3-[3-(4-chlorophenylsulphonyl)-propan-1-yl]-phenoxyacetic acid ;
3-[3-(4-bromophenylsulphonyl)-propan-1-yl]-phenoxyacetic acid ;
4-[3-(4-chlorophenylsulphonyl)-propan-1-yl]-phenoxyacetic acid
or
3-[4-(4-chlorophenylsulphonyl)-butan-1-yl]-phenoxyacetic acid.

4. The compounds
4-[3-(2-Methylphenylsulphonyl)-propan-1-yl]-phenylacetic acid
4-[3-(2-chlorophenylsulphenyl)-propan-1-yl]-phenylacetic acid

4-[3-(2-chlorophenylsulphonyl)-propan-1-yl]-phenylacetic acid
4-[3-(2-methylphenylsulphinyl)-propan-1-yl]-phenylacetic acid
4-[3-(3-chlorophenylsulphinyl)-propan-1-yl]-phenylacetic acid
4-[3-(3-chlorophenylsulphonyl)-propan-1-yl]-phenylacetic acid
4-[3-(3-trifluoromethylphenylsulphonyl)-propan-1-yl]-phenylacetic acid
4-[4-(2-chlorophenylsulphinyl)-butan-1-yl]-phenylacetic acid
4-[4-(2-chlorophenylsulphonyl)-butan-1-yl]-phenylacetic acid
4-[4-(3-bromophenylsulphinyl)-butan-1-yl]-phenylacetic acid
4-[4-(3-bromophenylsulphonyl)-butan-1-yl]-phenylacetic acid
4-[4-(2-methylphenylsulphenyl)-butan-1-yl]-phenylacetic acid
4-[4-(2-methylphenylsulphinyl)-butan-1-yl]-phenylacetic acid
4-[4-(2-methylphenylsulphonyl)-butan-1-yl]-phenylacetic acid
4-[4-(3-methylphenylsulphinyl)-butan-1-yl]-phenylacetic acid
4-[4-(4-cyanophenylsulphonyl)-butan-1-yl]-phenylacetic acid
4-[4-(4-methoxyphenylsulphenyl)-butan-1-yl]-phenylacetic acid
6-4-[3-(4-chlorophenylsulphenyl)-propan-1-yl]-phenyl-hexanoic acid
or
6-4-[3-(4-chlorophenylsulphonyl)-propan-1-yl]-phenyl-hexanoic acid.

5. Process for the preparation of oxyacetic acids according to claim 1, 2 or 3, characterised in that one reacts a compound of the formula IX

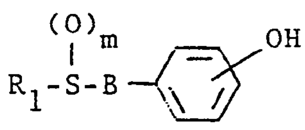

(IX)

in which $R_1$, B and m have the above-given meaning, with chloro- or bromoacetic acid or its esters and subsequently possibly converts the compounds obtained of the formula I into other compounds of the formula I in per se known manner or converts the free acids obtained into their pharmacologically compatible salts, esters or amides or the esters or amides obtained into the free acids.

6. Process for the preparation of compounds according to claim 1, 2, 3 or 4, characterised in that, in per se known manner, one reacts a compound of the formula II

X-B-W-A (II)

in which B, W and A have the above-given meaning and X represents a reactive residue, either with a mercaptan of the formula III

$R_1$-SH (III)

or with a sulphinic acid of the formula VI or one of its salts

$R_1$-SO-OH (IV)

in which $R_1$ has the given meaning, and subsequently possibly oxidises the sulphenyl compound obtained to the sulphinyl or sulphonyl compound or reduces the sulphonyl compound obtained to the sulphinyl or sulphenyl compound or reduces the sulphinyl compound obtained to the sulphenyl compound.

7. Process for the preparation of compounds according to claim 1 or 2, the $R_1$ of which represents an alkyl, alkenyl, cycloalkyl or aralkyl radical, characterised in that one reacts a mercaptan of the formula IV

HS-B-W-A (IV)

with a compound of the formula V

$R_1$-X (V)

whereby A, B, $R_1$, W and X have the given meaning, and subsequently possibly converts the compounds

obtained of the formula I into other compounds of the formula I in per se known manner or converts the free acids obtained into their pharmacologically compatible salts, esters or amides or the esters or amides obtained into the free acids.

8. Medicaments containing a compound according to claim 1, 2, 3 or 4, besides usual carrier and adjuvant materials.

9. Use of compounds according to claim 1, 2, 3 or 4 for the production of medicaments for the treatment of metabolic diseases.

10. Use of compounds according to claim 1, 2, 3 or 4 for the production of medicaments for the treatment of circulatory disturbances.